# EUROPEAN PATENT SPECIFICATION

(11) **EP 1 831 186 B1**
(45) Date of publication and mention of the grant of the patent: **28.07.2010**
(21) Application number: 05815283.6
(22) Date of filing: 29.11.2005
(51) Int. Cl.: C07D 257/04

(54) **A PROCESS FOR THE SYNTHESIS OF VALSARTAN**
VERFAHREN ZUR HERSTELLUNG VON VALSARTAN
PROCÉDÉ DE PRÉPARTION DE VALSARTAN

(30) Priority: 30.11.2004 EP 04028356
(43) Date of publication of application: 12.09.2007
(73) Proprietor: KRKA, D.D., Novo Mesto, 8501 Novo mesto (SI)
(72) Inventor: ZUPANCIC, Silvo, 8000 Novo mesto (SI); PECAVAR, Anica, 8000 Novo mesto (SI); ZUPET, Rok, 1211 Ljubljana (SI)
(74) Representative: HOFFMANN EITLE
(86) International application number: PCT/EP2005/012736
(87) International publication number: WO 2006/058701

(56) References cited:
- WO-A-97/30036
- WO-A-03/089417
- WO-A-2004/026847
- WO-A-2004/083192
- WO-A-2004/094391
- WO-A-2004/094392
- WO-A-2004/101534
- CARINI D J ET AL: "NONPEPTIDE ANGIOTENSIN II RECEPTOR ANTAGONISTS: THE DISCOVERY OF A SERIES OF N-(BIPHENYLYLMETHYL)IMIDAZOLES AS POTENT, ORALLY ACTIVE ANTIHYPERTENSIVES" JOURNAL OF MEDICINAL CHEMISTRY, AMERICAN CHEMICAL SOCIETY. WASHINGTON, US, vol. 34, no. 8, 1 August 1991 (1991-08-01), pages 2525-2547, XP000674205 ISSN: 0022-2623
- MOENIUS T ET AL: "Carbon-14 Labelling of DIOVAN in its Valine-Moiety" JOURNAL OF LABELLED COMPOUNDS AND RADIOPHARMACEUTICALS, SUSSEX, GB, vol. 43, 2000, pages 1245-1252, XP002292105 ISSN: 0362-4803

## Description

### Field of the invention

The present invention relates to a process for the manufacture of valsartan and pharmaceutically acceptable salts thereof as an active ingredient of a medicament for the treatment of hypertension and related diseases and conditions.

### Technical problem

Valsartan of formula (I) is chemically described as *N-*(1-oxopentyl)-*N-*{4-[2-(1H-tetrazole-5-yl)phenyl]-benzyl}-*L-*valine. Because of its ability to inhibit the angiotensin-converting enzyme it is widely used for the treatment of hypertension and related diseases and conditions. As an angiotensin II receptor antagonist, valsartan avoids the side-effects of calcium antagonists, and shows high stability and obvious curative effects.

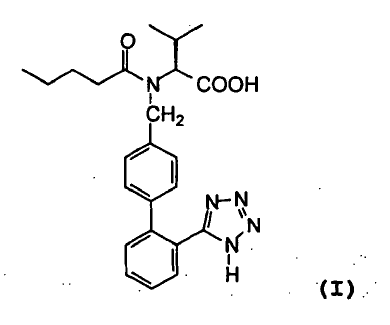

As is indicated herein below, it would be of great benefit to provide a process for providing valsartan and valsartan-containing formulations with a higher yield and better purity than existing processes.

### Background of the Invention

EP-B-0 443 983**,** Example 55, discloses a process for the preparation of valsartan benzyl ester wherein 2'-tetrazolylbiphenyl-4-carbaldehyde is reacted with (L)-valine benzyl ester hydrochloride in the presence of sodium cyanoborohydride. The obtained *N-*[(2'-tetrazolylbiphenyl-4-yl)-methyl]-(L)-valine benzyl ester is then acylated with valeryl chloride. In example 54 of this patent, the benzyl ester group is removed by catalytic hydrogenation and valsartan is obtained. By contrast, most of the other examples involve the use of a cyanobiphenyl intermediate to prepare valsartan analogues (sartans).

WO 2004/026847 relates to a process for the manufacture of valsartan using the same type of a reductive amination reaction in the first step with the difference that 2'-(1*H-*tetrazol-5-yl)biphenyl-4-carbaldehyde or various 2'-(1*H-*tetrazol-5-yl)-biphenyl-4-carbaldehydes having a protective group on the tetrazole nitrogen are used instead of the 2'-cyanobiphenyl-4-carbaldehyde. In other words, the formation of the (optionally protected) tetrazole moiety is carried out before the N-acylation step.

In »Synthesis of antihypertensive drug valsartan« in Zhongguo Yiyao Gongye Zazhi (2001), 32(9), 385-387, the valsartan synthesis starts with a substitution of 4'-bromomethyl-2-cyanobiphenyl with *L-*valine benzyl ester *p*-toluenesulfonate in DMF, the resulting compound is acylated with valeryl chloride in dichloromethane in the presence of diisopropylethylamine and subsequently hydrogenated in methanol in the presence of Raney Ni to remove the protective benzyl group. The tetrazole moiety is then formed with NaN₃ in butanol in the presence of ZnCl₂ and valsartan isolated with moderate to good yields. In particular, the last two steps are reported to have resulted in increased yields (40%).

In CN 1317485 valsartan is prepared via the acylation of *N-*[(2'-cyano[1,1'-biphenyl]-4-yl)methyl]-*L-*valine ester hydrochloride salt with pentanoyl chloride in an aromatic solvent in the presence of 4-dimethylaminopyridine and carbonate as catalysts, forming the corresponding pentanoyl ester. Further cyclization with sodium azide in the presence of trialkyltin chloride and polyoxyethylene bis(trimethylsilyl) ether as catalysts yields the product with 55% to 78%.

WO 03/089417 disloses polymorphic forms I and II of valsartan, and WO 2004/083192 teaches the preparation of polymorphic forms I-XIII as well as of substantially amorphous valsartan.

WO 2004/094391, Examples 1 and 2, discloses a process for the preparation of valsartan methyl ester wherein 5-(4'bromomethylbiphenyl-2-yl)-1-trityl-1H-tetrazole is reacted with (L)-valine methyl ester in acetonitrile. The obtained *N-*[(2'-tetrazolylbiphenyl-4-yl)-methyl]-(*L*)-valine methyl ester is then acylated with valeryl chloride. The combined yield of these two steps is reported to be 80%. The trityl group is removed by acid hydrolysis (acetone/HCl) and the methyl group is then removed by alkaline hydrolysis (aq. NaOH/MeOH), to thus obtain free valsartan.

### Summary of the Invention

In one aspect, the present invention relates to a process for preparing valsartan having the following formula (I), or a pharmaceutically acceptable salt thereof, or a pharmaceutical preparation containing either entity 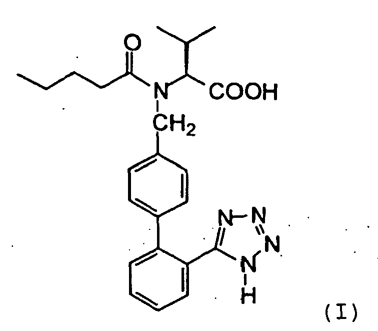 which process comprises the following steps:
(a) reacting a compound of formula (II) 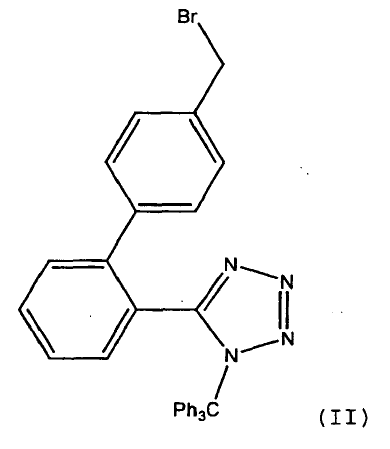 or salt thereof, with a compound of formula (III) 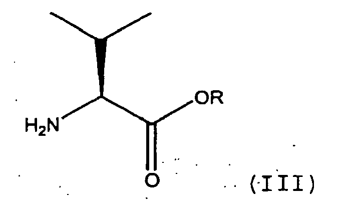 or a salt thereof, wherein R is a group selected from hydrogen, C₁₋₅ alkyl, C₂₋₅ alkenyl and benzyl which may optionally be substituted with one to three substituents selected from halogen atoms, C₁₋₅ alkyl, C₂₋₅ alkenyl, hydroxy, or nitro,
   in methylene chloride ;
(b) acylating the resulting compound of formula (IV) 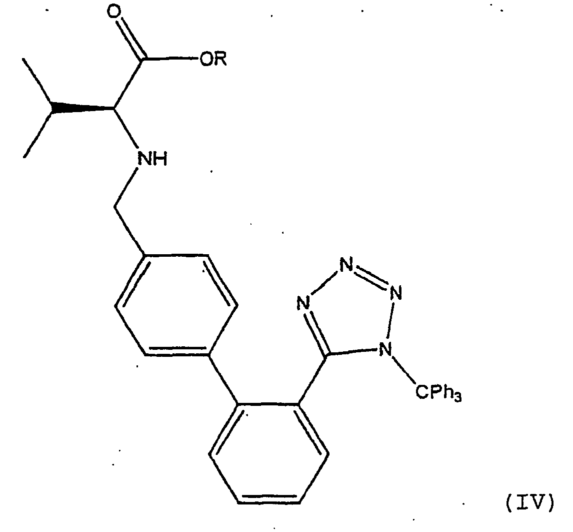 wherein R has the above meaning, or a salt thereof, with a compound of formula (V) 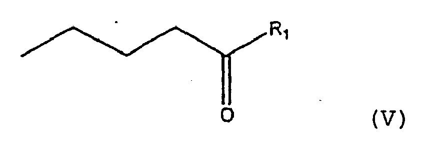 wherein R₁ is an activating group;
(c) removing the protecting group(s) in the resulting compound of formula (VI) 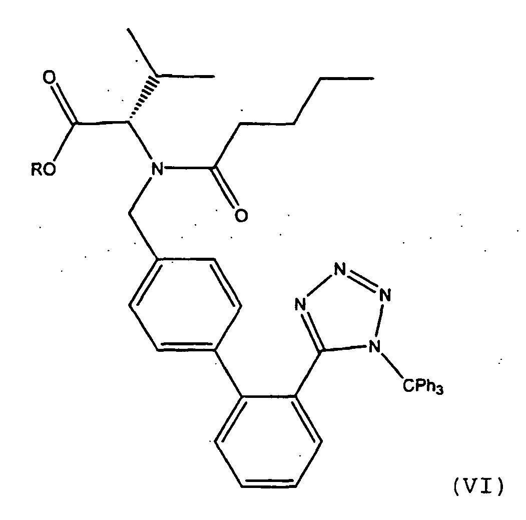 or a salt thereof, wherein R has the above meaning; and
(d) isolating the compound of formula (I), i.e. valsartan, or a pharmaceutically acceptable salt thereof, and
(e) if desired, formulating valsartan or its pharmaceutically acceptable salt into a pharmaceutical formulation by conventional means.

In step (d), the resulting compound (I) can be isolated in the amorphous form or in a crystalline or mixed crystalline form. Moreover, the substance can be isolated in any of its polymorphous forms. The isolation may include a post-treatment step such as melting and milling of solid valsartan.

One of the important advantages of the invention rests in the preparation of the intermediate (IV) with higher yields and improved purity compared to other synthetic routes known from the art.

The intermediate compound of formula (IV) 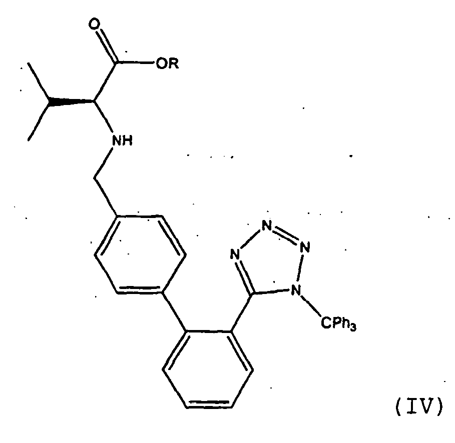 wherein R is hydrogen and the hydrochloride of a compound of formula (IV) wherein R is methyl are novel compounds and as such represent a further aspect of the present invention.

### Detailed Description of the Invention

In one aspect the present invention relates to a process for the manufacture of valsartan or a salt thereof, comprising:
step a: reacting a compound of formula (II) 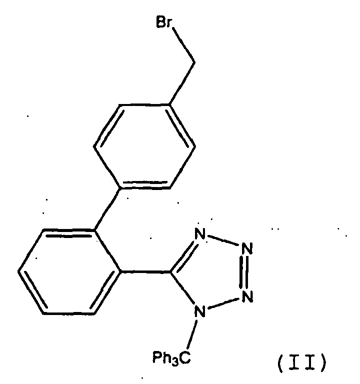 or salt thereof, with a compound of formula (III) 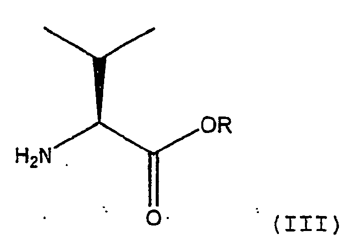 or a salt thereof, wherein R is selected from the group consisting of hydrogen, C₁₋₅ alkyl, C₂₋₅ alkenyl and benzyl which may optionally be substituted with one to three substituents selected from halogen atoms, C₁₋₅ alkyl, C₂₋₅ alkenyl, hydroxy, or nitro, and wherein R preferably represents hydrogen, methyl, ethyl or benzyl, more preferably methyl or benzyl and most preferably methyl, under conditions suitable for a nucleophilic substitution reaction in methylene chloride;
step b: acylating the resulting compound of formula (IV) 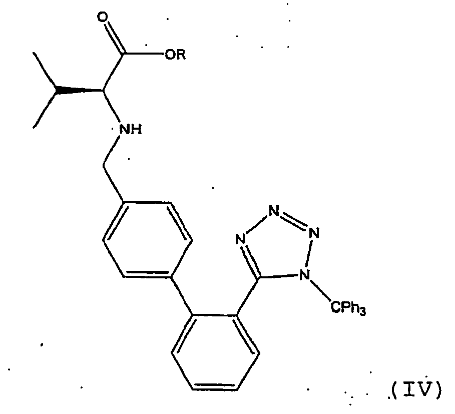 where R is as defined above, or a salt thereof with a compound of formula (V) 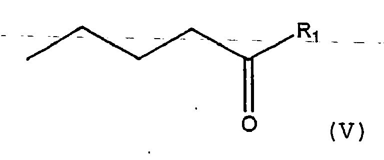 wherein R₁ is an activating group, preferably chlorine; and
step c: removing the protecting group(s) (i.e. the trityl group on the tetrazole ring and the ester group, if R is different from H) in the resulting compound of formula (VI) 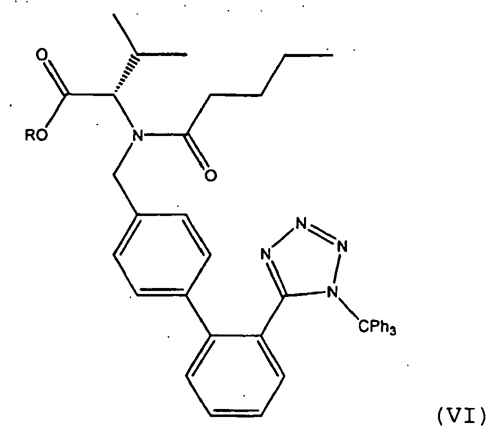 or a salt thereof; and
step d: isolating the resulting compound of formula (I) or a salt thereof in the amorphous form or in a crystalline or mixed crystalline form, e.g. in one of its polymorphous forms such as described in WO 03/089417 and WO 04/083192.

The valsartan or pharmaceutically acceptable salt thereof as obtained in step (d) may then be formulated to a pharmaceutical formulation (medicament) by conventional means, using appropriate pharmaceutically acceptable excipients (step e).

Known oral dosage forms of valsartan are described, e.g. in EP 0 914 119 and EP 1 410 797 as well as in WO 95/24921. The formulations as disclosed in EP-B-443 983 may also be used.

Pharmaceutical formulations of valsartan can generally be prepared by known technological procedures, e.g. wet water granulation or direct compression, using well known and readily available excipients. In preparation of the compositions of valsartan, the active ingredient will usually be mixed with an excipient or mixture of excipients, or diluted by an excipient or mixture of excipients, or enclosed within an excipient or mixture of excipients which may be in the form of a capsule, sachet, paper or other container. When the excipient serves as a diluent, it may be a solid, semisolid or liquid material which acts as a vehicle or medium for the active ingredient.

Thus, the compositions can be in the form of tablets, pills, powders, lozenges, sachets, cachets, elixirs, suspensions, emulsions, solutions, syrups, aerosols, ointments, soft and hard gelatine capsules, suppositories etc. The pharmaceutical composition contains valsartan or a pharmaceutically acceptable salt thereof as an active ingredient and excipient(s).

The compositions are preferably formulated in a unit dosage form, each dosage containing about 1 to about 1000 mg, more usually about 40 to about 320 mg of the active ingredient. The term »unit dosage form« refers to physically discrete units suitable as unitary dosages for human subjects and other mammals, each unit containing a predetermined quantity of active ingredient calculated to produce the desired therapeutic affect, in association with a suitable pharmaceutical excipient.

In step (a) R is preferably methyl, ethyl or hydrogen. However, R may also be a benzyl group. In the definition of R, C₁₋₅ alkyl means any group selected from methyl, ethyl, n-propyl, isopropyl, n-butyl, sec-butyl, tert-butyl, n-pentyl, isopentyl, sec-pentyl. The term C₂₋₅ alkenyl is to be construed analoguously and is preferably ethenyl and 1- or 2-propenyl. The benzyl group is preferably unsubstituted but may also be substituted by e.g. 1-3 chlorine or other halogen atoms or by 1-3 alkyl groups, e.g. by 1-3 methyl or ethyl groups. 2,4-dichlorobenzyl groups, 4-chlorobenzyl groups, 2,4-dimethylbenzyl groups, 2,3,4-trimethylbenzyl groups, 2,4-dinitrobenzyl groups and the like may be mentioned as examples of substituted benzyl groups.

In step b) the activating group R₁ can be an activating group as it is used in the field of peptide synthesis, such as halogen (particularly chlorine or bromine), C₁-C₇ alkylthio, pyridylthio, imidazolyl, benzthiazolyloxy, benzotriazoloxy, C₂-C₈-alkanoyloxy; or 2,5-dioxo-pyrrolidinyl-1-oxy. Most preferably, the activating group is chlorine.

The synthesis of valsartan according to a preferred embodiment of the present invention starts from 5-(4-(bromomethyl)-biphenyl-2-yl)-1-(triphenylmethyl)tetrazole (II) and *L*-valine methyl ester of formula (III). *L-*Valine methyl ester of formula (III) is a basic amino acid, and is known as a reagent also from Chemistry of the Amino Acids by J. P. Greenstein and M. Winitz, published by John Wiley and Sons, (1961). 5-(4-(Bromomethyl)biphenyl-2-yl)-1-(triphenylmethyl)-tetrazole (II) is likewise known from the synthesis of other sartans, such as losartan, and has been described e.g. in EP 0 253 310, or in D. J. Carini, J. V. Duncia, P. E. Aldrich , A. T. Chiu, A. L. Johnson, M. E. Pierce, W. A. Price, J. B. Santella III, G. J. Wells, R. R. Wexler, P. C. Wong, S.-E. Yoo, P. B. M. W. M. Timmermans, Nonpeptide Angiotensin II Receptor Antagonists: The Discovery of a Series of N-(Biphenylylmethyl)imidazoles as Potent, Orally Active Antihypertensives, J. Med. Chemistry, 1991, 34, 2525-2547.

In step a) the nucleophilic substitution reaction of mono *N-*alkyl *L-*valine methyl ester (III) and 5-(4-(bromomethyl)biphenyl-2-yl)-1-(triphenylmethyl)tetrazole (II), is carried out in methylene chloride, at any optional temperature. In a preferred embodiment the reaction is carried out under reflux. Preferably 3-6 equivalents of *L-*valine methyl ester are used in regard to 5-(4-(bromomethyl)biphenyl-2-yl)-1-(triphenylmethyl)tetrazole. *L*-valine methyl ester can be used in the form of a salt. The most preferred form of an *L-*valine methyl ester salt is the hydrochloride salt of *L*-valine methyl ester. *L-*valine methyl ester hydrochloride is a particularly preferred starting material according to the present invention.

If an acid addition salt of valine or a valine ester is used, the salt needs to be neutralized before the nucleophilic substitution reaction with 5-(4-(bromomethyl)biphenyl-2-yl)-1-(triphenylmethyl)tetrazole. This is conveniently carried out by adding an aqueous base such as aqueous NaOH. Excess valine or valine ester can be regenerated in a simple way. Alkylation is carried out in 4 to 22 h, in a preferred embodiment between 5 and 8 h, until all 5-(4-(bromomethyl)biphenyl-2-yl)-1-(triphenylmethyl)tetrazole has reacted and no dialkylated product (VII) has yet been detected.

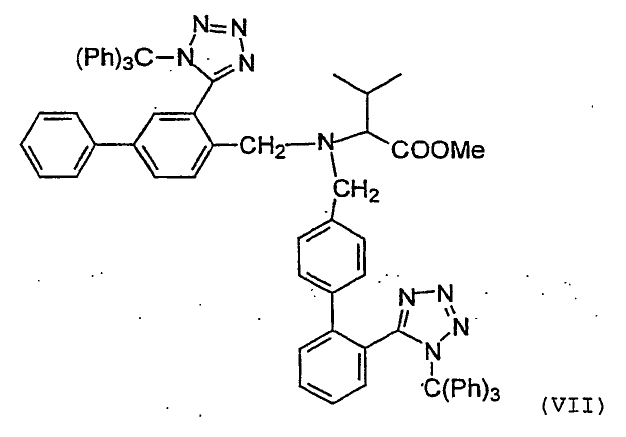

The resulting product, intermediate compound (IV), is isolated as an oily substance. The reaction mixture is cooled, diluted with an organic solvent such as ethers, esters, hydrocarbons and halogenated hydrocarbons, washed with a diluted aqueous acid solution and subsequently with water. The organic phase is separated and evaporable components are distilled off under reduced pressure. The first, acidic water phase can be used for the regeneration of *L*-valine methyl ester. The intermediate (IV) can also be isolated in the form of its hydrochloride after addition of HCl.

The yield of the transformation from compound (II) to (IV) is over 98 % and HPLC purity of the product is over 95 %.

The acylation of intermediate (IV) is then carried out in an organic solvent. Preferably such solvents are selected from tetrahydrofurane (THF), t-butyl methyl ether, methylene chloride, isopropyl acetate. Most preferred is THF. The most preferred acylating agent is valeryl chloride (pentanoyl chloride). Valeryl chloride is preferably used in excess. Preferably, more than 1 to 3 equivalents of valeryl chloride are used in regard to intermediate (IV). The reaction can be carried out in the presence of a basic substance that acts as a catalyst or a co-reactant, and a useful basic substance for the reaction is pyridine or another organic or inorganic base that is able to remove hydrogen chloride that will be formed as a by-product, such as DMAP and triethylamine (TEA). The reaction can be carried out at room temperature and will then be finished in 4 hours, preferably in 1 to 2 hours, more preferably in 2 hours or less. After that, the reaction mixture can be quenched, e.g. by addition of an aliphatic alcohol, for example methanol or ethanol.

The mixture is diluted with water and the product (VI) is extracted with an organic solvent such as an ester, ether, hydrocarbon and/or halogenated hydrocarbon. The organic phase is then washed, dried and evaporated.

Under the above preferred conditions, the yield of the acylation step is over 97 % and HPLC purity of the product is over 95 %.

Surprisingly, it was found out that the deprotection of the trityl (CPh₃) group of compound (VI) can advantageously be carried out in refluxing alcohol by addition of a mineral base. The by-product of this reaction is alkyl trityl ether. However, deprotection can also be achieved as taught in "Protective Groups in Organic Synthesis" by T. W. Greene and P. G. M. Wuts, published by John Wiley and Sons (1981) or in Protective Groups in Organic Chemistry edited by J. F. McOmie, published by Plenum Press. The term alcohol as used herein means a C₁-C₄ alcohol such as methanol, ethanol, n-propanol, iso-propanol and n-butanol and most preferably ethanol. While the deprotection is preferably carried out at elevated temperatures, preferably at the reflux temperature of the solution, the reaction can also be carried out at lower temperatures, e.g. at room temperature.

In a particularly advantageous variant of the present invention, compound (VI) is dissolved in alcohol such as methanol, ethanol, isopropanol, n-butanol or 2-butanol and then a solid mineral base such as KOH is added (1 to 5 equivalents) to effect the deprotection reaction. The reaction is carried out at temperature from room temperature to the reflux temperature of the alcohol in 1 to 8 hours. When the removal of the trityl group is completed, isolation of the product is not necessary. If the starting material of the reaction sequence was an *L-*valine alkyl or alkenyl ester, it is sufficient that an aqueous mineral base (e.g. NaOH, KOH, LiOH etc., in an amount of 1 to 5 equivalents) is added to the reaction mixture and the reaction mixture is further heated for 1 to 6 hours under stirring, or is simply stirred at room temperature for several hours. After the hydrolysis of the ester group has been completed, the isolation of valsartan is performed. The reaction mixture is cooled, diluted with water and then the alcohol is evaporated. The residue is extracted with an organic solvent such as an ether, ester, hydrocarbon and/or halogenated hydrocarbon. Then the water phase is acidified with a mineral acid to pH below 3 and again extracted with an organic solvent such as an ether, ester, hydrocarbon and/or halogenated hydrocarbon. The organic phase is then washed with water, dried and evaporated. In the case that R is an optionally substituted benzyl group, such benzyl group may also be removed by catalytic hydrogenation as is known to one skilled in the art.

Under the above conditions, the yield of the transformation from compound (VI) to valsartan is over 94 % and HPLC purity of the product is over 95 %.

The overall yield of the synthesis of valsartan starting from compound (II) with R=methyl is over 90 % and HPLC purity of the product is over 95 %.

The preparation of valsartan from compound (VI) is also possible by first hydrolyzing the methyl ester of intermediate (IV) in DMSO as a solvent by addition of an aqueous solution of NaOH. Then, after re-acidification, the trityl protective group is removed and valsartan is isolated by means of extraction with *tert-*butyl methyl ether or another organic solvent such as an ether, ester, hydrocarbon and/or halogenated hydrocarbon.

The extract of valsartan can further be processed in any of the following ways:
a. Evaporation of the organic solvent.
b. Spray-drying of the valsartan solution. Any related method for the preparation of amorphous valsartan can be used as well.
c. Melting and re-cooling of valsartan and optionally milling it to the amorphous form.
d. Crystallization from ethyl acetate or other organic solvents such as propyl acetate, isopropyl acetate, methylene chloride.

Alternatively, the valsartan extract can further be purified before any of the above steps. Moreover, steps a and c, or steps b and c can be used in combination.

If desired, valsartan can be crystallized, e.g. in the presence of dicalite (diatomaceous earth). The suspension obtained shows improved filterability if dicalite is added. Dicalite is removed from the precipitate in such a way that valsartan is dissolved in an aqueous alkaline solution, the remaining dicalite is separated off by filtration and valsartan is then precipitated out by the addition of an acid to the alkaline solution of valsartan, until all valsartan has precipitated from the solution. Specific polymorphous forms of valsartan can also be prepared according to the teachings of WO 2003/089417 and WO 2004/083192.

In another preferred embodiment, a salt of valsartan can be prepared in order to achieve purification, such as a valsartan salt with an alkali or earth alkaline metal, an amine salt with NR₁R₂R₃, wherein R₁, R₂, and R₃ can be chosen among C₁-C₈ alkyl and C₆-C₁₀ aryl groups, or a salt with any other basic compound. Such valsartan salts as used for the purification need not necessarily be pharmaceutically acceptable, provided that they are later converted back into valsartan free base or a pharmaceutically acceptable salt of valsartan. Pharmaceutically acceptable salts of valsartan include any non-toxic and pharmaceutically acceptable salts of valsartan and may be either alkaline salts (e.g. the sodium or potassium salt or a calcium or magnesium salt) or acid addition salts such as the hydrochloride, oxalate, citrate, acetate, lactate etc. As a (pharmaceutically acceptable) salt, the hydrochloride is generally preferred in this invention.

The valsartan used as a starting material in these purification processes can be in any form, e.g. it can be in reaction solution, crude, in filtrate, in an anhydrous, solvated, or hydrated form, or in a mixture of any of these forms.

When evaporation of the solvent is applied, valsartan is dissolved in an organic solvent having a boiling point below 100°C in which it is well soluble. Suitable examples of such solvents include methanol, ethanol, isopropanol, methyl acetate, ethyl acetate, isopropyl acetate, acetonitrile, methylene chloride, *tert-*butyl methyl ether, tetrahydrofurane, acetone and methyl ethyl ketone or their mixtures or mixtures with water. The dissolution of valsartan can be achieved at any temperature from room temperature to the temperature of the boiling point of the solution. After a solution has been obtained, the solvent is evaporated under vacuum or optionally at atmospheric pressure or a combination thereof at temperatures ranging from boiling point of the solution to -20°C to isolate valsartan. The solid or oily residue is collected and dried and amorphous valsartan is obtained.

When spray-drying of the solvent is applied, valsartan is dissolved in an organic solvent having a boiling point below 100°C in which it is well soluble. Suitable examples include methanol, ethanol, isopropanol, methyl acetate, ethyl acetate, isopropyl acetate, acetonitrile, methylene chloride, *tert-*butyl methyl ether, tetrahydrofurane, acetone and methyl ethyl ketone or their mixtures or mixtures with water. The dissolution of valsartan can be achieved at any temperature from room temperature to the boiling point of the solution. The concentration of valsartan is not particularly limited and depends only on the solubility of valsartan in the chosen solvent. The solution is sprayed in a flow of hot gas in a suitable apparatus such as a Spray Dryer Büchi 190. By this process the solvent evaporates from the solution and solid amorphous valsartan precipitates. Gases applied for drying can be chosen from nitrogen, argon, carbon dioxide or air, or their mixtures. The temperature of the hot air can be optional and depends on the temperature of the boiling point of the solvent used for the preparation of the solution of valsartan. After the separation a pure amorphous product is obtained which can optionally be additionally dried.

Instead of spray-drying other methods for the preparation of solid amorphous valsartan can also be used such as lyophilisation etc.

Uniformly amorphous valsartan can also be prepared by melting valsartan in mills usually used for pharmaceuticals and by quenching the melt, optionally in liquid nitrogen. In another preferred embodiment, the cooling is carried out slowly.

Amorphous, crystalline or mixed crystalline valsartan can be isolated from solvents such as esters (methyl acetate, ethyl acetate, ethyl formate, propyl acetate, isopropyl acetate, propyl formate, isopropyl formate, isobutyl acetate), methylene chloride, chloroform, 2-butanone, or mixtures thereof. The isolation of amorphous, crystalline or mixed-crystalline valsartan can also be performed from mixtures of above solvents with apolar solvents such as hexane, heptane, cyclohexane, toluene, anisole or petrol ether.

Crude valsartan is dissolved in any of the above solvents at a higher temperature, filtered, then cooled and the product is isolated. The thus obtained valsartan can be treated with apolar solvents to remove residual solvent (trituration of the product in an apolar solvent, evaporation of a part of the solvent at lower pressure and filtration of the product). Depending on the exact process conditions applied, purified valsartan will be obtained in either amorphous, crystalline or mixed crystalline form.

Valsartan can be dried at temperatures between 0°C and 70°C, preferably 0°C and 60°C, most preferably between 40°C and 60°C using usual drying methods. Such drying methods can be applied following the above-described solvent evaporation or isolation steps.

A preferred drying process according to the present invention comprises the following steps after the isolation step(s):
(a) drying the valsartan or its pharmaceutically acceptable salt at room temperature,
(b) during or after step (a) milling or granulating the substance to an average particle size of 100 µm or less
(c) drying the substance at elevated temperature under reduced pressure to a solvent content of 5000 ppm or less.

In a particularly preferred embodiment of this process (as well as in the preceding solvent evaporation or isolation process), the solvent is ethyl acetate.

The following drying conditions are particularly preferred:
- the solvent content (e.g. the ethyl acetate content) after step (a) is between 5 and 20% by weight. This can be achieved, e.g. by drying the substance collected from the filter or from a centrifuge under air at room temperature, or by drying the substance in a vacuum drier at room temperature with a slight air flow.
- the particle size distribution (as determined by electron microscopy) at the end of step (b) is so narrow that the number of particles having a diameter of more than 150 µm is minimal (i.e. less than 1% of the particles). This can be achieved by granulating or milling the particles to reduce the size of the particle structures and agglomerates.
- the drying temperature in step (c) is between 40 and 60°C
- the drying time in step (c) is 36h or less, preferably 24h or less
- the pressure in step (c) is 150 hPa (mbar) or less. The drying in step (c) can be carried out in a sealed vacuum drier or a rotary vacuum drier at a pressure most preferably between 40 and 100 hPa (mbar). Alternatively, drying can also be carried out in a vacuum drier or in a rotary vacuum drier at a pressure of 150 hPa (mbar) or less at a temperature between 40 and 60°C with a slight gas flow. The gas can be air, nitrogen, argon and helium. the solvent content at the end of step (c) is below 5000 ppm.

The most preferred drying process of the present invention fulfills each of the following conditions, but some deviations particularly as regards the drying temperature, time and pressure are also possible. A skilled person will readily understand that these parameters are mutually interdependent.

The invention is further illustrated by the following examples. Parts or percentages are based on weight, unless specified otherwise. However, yields are indicated in mol%.

### Examples

### Example 1

### Preparation of methyl (S)-3-methyl-2-((2'-(1-triphenylmethyl-tetrazol-5-yl)-biphenyl-4-ylmethyl)amino) butanoate (IV)

1.37 g (8.2 mmol) of *L-*valine methyl ester hydrochloride are dissolved in 8.8 ml aqueous 1M NaOH and 8.5 ml of methylene chloride are added and mixed at room temperature. The phases are separated, the organic phase is filtered over Na₂SO₄ and washed with 10 ml of methylene chloride. 1.0 g (1.8 mmol) 5-(4-(bromomethyl)biphenyl-2-yl)-1-(triphenylmethyl)tetrazole is added to the filtrate and the mixture is heated under reflux for 20 h. After the completion of the reaction the mixture is cooled to room temperature, diluted with 16.5 ml of methylene chloride and washed with 16.5 ml of 1% HCl. The organic phase is washed (2 × 16.5 ml of water), dried over Na₂SO₄, filtered and evaporated. 1.1 g of the product in the form of a viscous oil are obtained (HPLC Area %: 97.3 %, yield: 100 %).

### Preparation of methyl (S)-3-methyl-2-((2'-(1-triphenylmethyl-tetrazole-5-yl)-biphenyl-4-ylmethyl)amino) butanoate hydrochloride

The above product is dissolved in ethyl acetate and then 0.33 ml 5M HCl are added. The mixture is stirred at 0°C for 2 h. The solid product is collected by filtration and then dried.
m.p.: 175 - 181°C
IR: 1750, 1569, 1449, 1441, 1213, 844, 758, 752, 746, 696 cm⁻¹

### Regeneration of excess L-valine methyl ester

The first water phase obtained in the isolation of the product when washing the organic phase with 1% HCl is made alkaline by means of 1M NaOH, and *L-*valine methyl ester is extracted into methylene chloride. The obtained *L-*valine methyl ester solution can be reused for a further reaction.

### Example 2

### Preparation of methyl (S)-3-methyl-2-(pentanoyl-(2'-(1-triphenylmethyltetrazole-5-yl)-biphenyl-4-ylmethyl)amino) butanoate (VI)

1.1 g (1.8 mmol) of methyl (S)-3-methyl-2-((2'-(1-triphenylmethyltetrazol-5-yl)biphenyl-4-ylmethyl)amino) butanoate prepared in example 1 are dissolved in 5.7 ml of dry tetrahydrofurane. 0.35 ml of pyridine and 0.4 ml of valeryl chloride (3.4 mmol) are added and the mixture is stirred under an inert gas atmosphere for 1.5 h. After the completion of the reaction 0.25 ml of methanol are added and the reaction mixture is stirred for 10 minutes. The mixture is diluted with 7 ml of water and the product is extracted with *tert*-butyl methyl ether (2 × 5 ml). The combined organic phases are washed with 3 × 7 ml of water and dried over Na₂SO₄, filtered and evaporated. 1.2 g of product in the form of a viscous oil are isolated (HPLC Area %: 97.4 %, yield: 97 %). After a while the oil is crystallized and is triturated with hexane. The solid product is filtered and dried.
m.p. = 100 - 103°C
IR: 1744, 1666, 1435, 1199, 755, 700 cm⁻¹

The combined yield of Examples 1 and 2, based on compound (II), i.e. 5-(9-(bromomethyl)biphenyl-2-yl)-1-(triphenylmethyl)tetrazole, is about 94%. By comparison, the combined yield of Examples 1 and 2 of WO 2004/094391 that use a different solvent system is reported to be only 80%.

### Example 3

### Preparation of crude valsartan (I)

1.2 g of methyl (S)-3-methyl-2-(pentanoyl-(2'-(1-triphenylmethyl-tetrazol-5-yl)-biphenyl-4-ylmethyl)amino) butanoate, prepared as in example 2, are dissolved in 10.7 ml of methanol and 0.16 g (2.9 mmol) of pulverized KOH are added. The mixture is first heated under reflux for 4 h, and then 2.5 ml 2M KOH are added. The mixture is then heated for another 5h under reflux. After the completion of the reaction the evaporable components are distilled off and the water phase is acidified to pH 1 and extracted with *tert-*butyl methyl ether (2 × 12 ml). The organic phase is washed twice with 16 ml of water, dried over Na₂SO₄ and evaporated to leave a dry residue. 0.72 g of crude valsartan are isolated (HPLC Area %: 98.0 %, yield: 95 %, yields based on compound (II) : 92 %).

By contrast, the yield in the deprotection step (Example 3 of WO 2004/094391) is only 81%.

### Example 4

### Purification and crystallization of valsartan

1g of crude valsartan is dissolved in 9 ml of ethyl acetate at a higher temperature. Then the solution is filtered and cooled to 0°C and stirred at this temperature for 1h. Pure valsartan is filtered off and dried under reduced pressure. 0.88 g of amorphous valsartan are isolated.

1g of crude valsartan is dissolved in 8 ml of ethyl acetate at higher temperature. Then the solution is filtered and cooled to 0°C and stirred at this temperature for 1h. Pure valsartan is filtered off and then 30 ml of heptane are added. 1/3 of the solvent is evaporated under reduced pressure at 30 to 40°C. The product is filtered off and dried at room temperature. 0.9 g of amorphous valsartan are isolated.

### Example 5

### Spray-drying of valsartan

5 g of valsartan are dissolved in 30 ml of acetone at room temperature. The solution is poured into a Büchi 190 spray dryer and drying is carried out at a gas temperature of 50°C for 15 minutes. 1.32 g of uniformly amorphous valsartan are obtained.

### Example 6

### Evaporation of a valsartan solution

0.5 g of valsartan are dissolved in 2 ml of acetone at room temperature. The evaporable components are distilled off under vacuum at 50°C. The residue is cooled, collected and additionally dried under vacuum at 45°C. Uniformly amorphous valsartan is obtained (90% yield).

### Example 7

### Evaporation of a valsartan solution

0.5 g of valsartan are dissolved in 4.3 ml of a mixture of ethyl acetate and water (12:1, v/v) at 50°C. The evaporable components are distilled off under vacuum at 50°C. The residue is cooled, collected and additionally dried under vacuum at 45°C. Uniformly amorphous valsartan is obtained (92 % yield).

### Example 8

### Evaporation of a valsartan solution

0.5 g of valsartan are dissolved in 4.5 ml of a mixture of methyl acetate and water (8:1, v/v) at room temperature. The evaporable components are distilled off under vacuum at 40°C. The residue is cooled, collected and additionally dried under vacuum at 45°C. Uniformly amorphous valsartan is obtained (92% yield) .

### Example 9

### Melting of valsartan

1 g of valsartan is heated in a round bottom flask at a temperature of 130°C and slowly cooled to room temperature after all valsartan has melted. Uniformly amorphous valsartan is obtained.

### Example 10

### Crystallization of valsartan from various solvents

### a) Crystallization from isopropyl acetate

0.5g of crude valsartan are dissolved is 1.5 ml of isopropyl acetate at a higher temperature. Then the solution is cooled to room temperature and stirred at this temperature for 1h. Pure valsartan is filtered off and dried under reduced pressure. 0.48 g of valsartan are isolated.

### b) Crystallization from propyl acetate

1 g of crude valsartan is dissolved in 3 ml of propyl acetate at a higher temperature. Then the solution is cooled to room temperature and stirred at this temperature for 1h. Pure valsartan is filtered off and dried under reduced pressure. 0.95 g of valsartan are isolated.

### c) Crystallization from methyl acetate

1 g of crude valsartan is dissolved in 2 ml of methyl acetate at a higher temperature. Then the solution is cooled to room temperature and stirred at this temperature for 1h. Pure valsartan is filtered off and dried under reduced pressure. 0.91 g of valsartan are isolated.

### d) Crystallization from isobutyl acetate

0.5 g of crude valsartan is dissolved in 3 ml of isobutyl acetate at higher temperature. Then the solution is cooled to 0°C and stirred at this temperature for 1h. Pure valsartan is filtered off and dried under reduced pressure. 0.49 g of valsartan are isolated.

### e) Crystallization from methylene chloride

1 g of crude valsartan is dissolved in 20 ml of methylene chloride at a higher temperature. Then the solution is cooled to room temperature and stirred at this temperature for 1h. Pure valsartan is filtered off and dried under reduced pressure. 0.88 g of valsartan are isolated.

### f) Crystallization from a mixture of isopropyl acetate / cyclohexane (1:1)

0.5 g of crude valsartan is dissolved in 4 ml of the mixture isopropyl acetate / cyclohexane (1:1) at a higher temperature. Then the solution is cooled to room temperature and stirred at this temperature for 1h. Pure valsartan is filtered off and dried under reduced pressure. 0.45 g of valsartan is isolated.

## Claims

1. A process for preparing valsartan having the following formula (I) or a pharmaceutically acceptable salt thereof, or a pharmaceutical preparation containing either entity 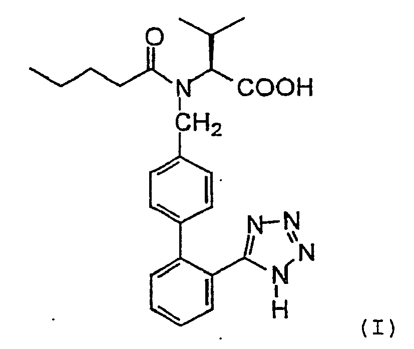 which process comprises the following steps:
(a) reacting a compound of formula (II) 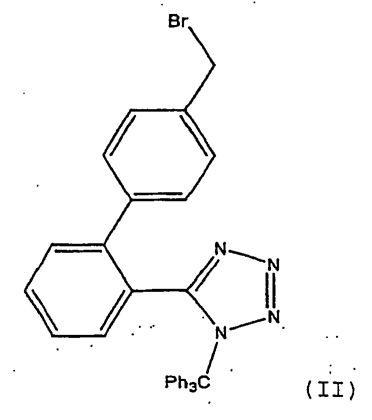 or salt thereof, with a compound of formula (III) 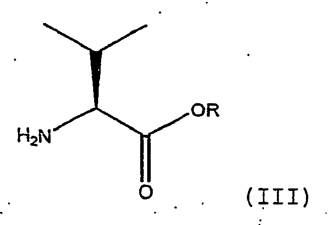 or a salt thereof, wherein R is selected from the group consisting of hydrogen, C₁₋₅ alkyl, C₂₋₅ alkenyl and benzyl which may optionally be substituted with one to three substituents selected from halogen atoms, C₁₋₅ alkyl, C₂₋₅ alkenyl, hydroxy, or nitro, to form a compound of formula (IV)
in methylene chloride ;
(b) acylating the resulting compound of formula (IV) 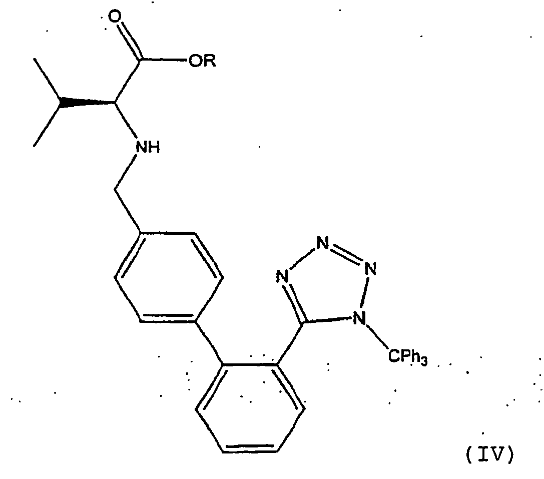 wherein R has the above meaning, or a salt thereof with a compound of formula (V) 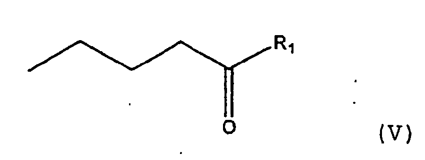 wherein R₁ is an activating group;
(c) removing the protecting group(s) in the resulting compound of formula (VI) 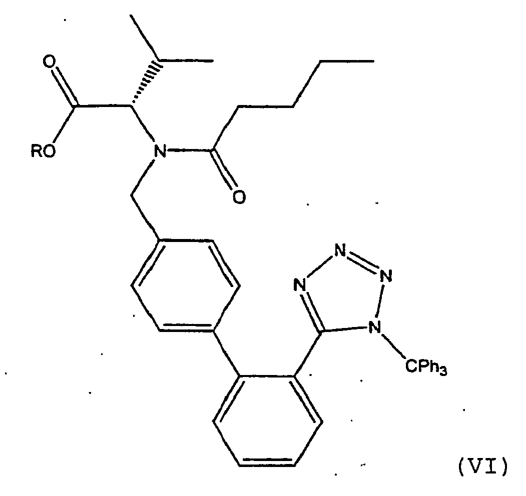 or a salt thereof, wherein R has the above meaning; and
(d) isolating the compound of formula (I), i.e. valsartan, or a pharmaceutically acceptable salt thereof, and
(e) if desired formulating valsartan or its pharmaceutically acceptable salt into a pharmaceutical formulation by conventional means.

2. A process according to claim 1 wherein R is methyl and the activating group R₁ is chlorine.

3. A process according to any of the preceding claims wherein compound (IV) is isolated in step (a) as a hydrochloride.

4. A process according to any of the preceding claims wherein step (a) is carried out in the presence of an excess of compound (III) or a salt thereof, and unused compound (III) is regenerated.

5. A process according to any of the preceding claims wherein step (b) is conducted in a solvent selected from THF, t-butyl methyl ether, methylene chloride and isopropyl acetate.

6. A process according to claim 5, wherein the solvent is THF.

7. A process according to any of the preceding claims wherein step (b) is conducted in the presence of a basic substance selected from pyridine, dimethylaminopyridine (DMAP) and triethyl amine (TEA).

8. A process according to any of the preceding claims wherein the reaction time of step (b) is 2 hours or less and the reaction is then quenched with methanol or ethanol.

9. A process according to any of the preceding claims wherein the removal of the CPh₃ group in step (c) is effected in alcohol with the addition of a mineral base at a temperature from room temperature to the reflux temperature of the reaction solution.

10. A process according to claim 1 wherein
- R is methyl or benzyl,
- the activating group is chlorine
- the solvent in step (b) is THF
- step (b) is carried out in the presence of a basic substance selected from pyridine, dimethylaminopyridine (DMAP) and triethyl amine (TEA)
- in step (c) the removal of the CPh₃ group in step (c) is effected in alcohol with the addition of a mineral base at a temperature from room temperature to the reflux temperature of the reaction solution.

11. A process according to any of the preceding claims wherein in step (d) valsartan or its pharmaceutically acceptable salt is isolated in a form selected from (i) an amorphous form, (ii) a crystalline or (iii) a mixed crystalline form.

12. A process according to claim 11 **characterized in that** valsartan or a pharmaceutically acceptable salt thereof is isolated from a valsartan-containing solution in its amorphous form by evaporation of the organic solvent.

13. A process according to claim 11 **characterized in that** valsartan or a pharmaceutically acceptable salt thereof is isolated from a valsartan-containing solution in its amorphous form by spray-drying of the valsartan-containing solution.

14. A process according to claim 12 or claim 13 wherein, following the evaporation or spray-drying step, the valsartan or pharmaceutically acceptable salt thereof is melted and cooled again, optionally followed by milling the cooled substance to yield the amorphous form of valsartan.

15. A process according to claim 11 comprising the step of isolating valsartan or its pharmaceutically acceptable salt in an amorphous, crystalline or mixed crystalline form from a solvent selected from methyl acetate, ethyl acetate, ethyl formate, propyl acetate, isopropyl acetate, propyl formate, isopropyl formate, isobutyl acetate, methylene chloride, chloroform or mixtures thereof, optionally in admixture with an apolar solvent selected from hexane, heptane, cyclohexane, toluene, anisole or petrol ether.

16. A process according to claim 12 or 15 which, after the isolation step(s), further comprises the steps of
(a) drying the valsartan or its pharmaceutically acceptable salt at room temperature,
(b) during or after step (a) milling or granulating the substance to an average particle size of 100 µm or less
(c) drying the substance at elevated temperature under reduced pressure to a solvent content of 5000 ppm or less.

17. Process according to claim 16 wherein the solvent is ethyl acetate.

18. Process according to claim 16 or 17 in which at least one of the following conditions apply:
- the solvent content after step (a) is between 5 and 20% by weight,
- the particle size distribution at the end of step (b) is so narrow that the number of particles having a diameter of more than 150 µm is minimal
- the drying temperature in step (c) is between 40 and 60°C
- the drying time in step (c) is 36h or less, preferably 24h or less
- the pressure in step (c) is 150 hPa or less, most preferably between 40 and 100 hPa
- the solvent content at the end of step (c) is below 5000 ppm.

19. A compound of formula (IV) or a salt thereof, wherein R is hydrogen

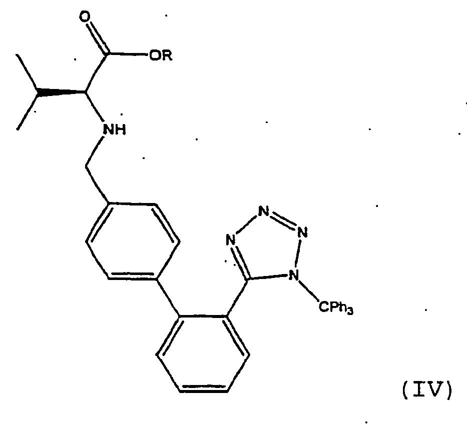

20. A solid compound representing the hydrochloride of the compound of formula (IV), wherein R is methyl

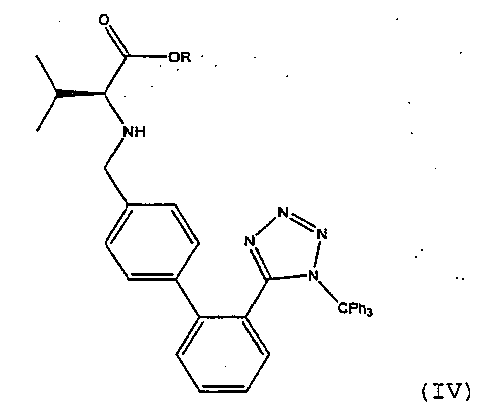

21. Use of a compound according to any of the claims 19 or 20 for the synthesis of valsartan or a pharmaceutically acceptable salt thereof.

## Patentansprüche

1. Verfahren zur Herstellung von Valsartan mit der folgenden Formel (I) oder eines pharmazeutisch akzeptablen Salzes davon oder einer pharmazeutischen Zubereitung, die eine dieser beiden Verbindungen enthält 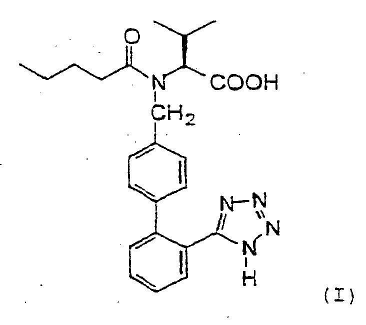 wobei das Verfahren die folgenden Schritte umfasst:
(a) Umsetzung einer Verbindung der Formel (II) 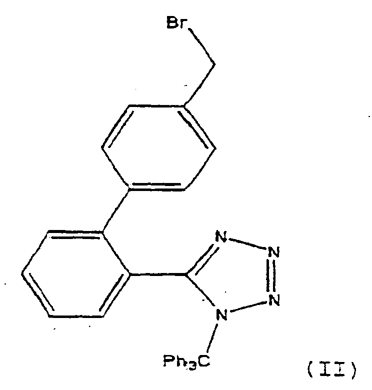 oder eines Salzes davon, mit einer Verbindung der Formel (III) 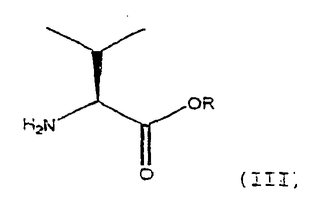 oder eines Salzes davon, worin R ausgewählt ist aus der Gruppe bestehend aus Wasserstoff, C₁₋₅-Alkyl, C₂₋₅-Alkenyl und Benzyl, das wahlweise substituiert sein kann mit einem bis drei Substituenten, ausgewählt aus Halogenatomen, C₁₋₅-Alkyl, C₂₋₅-Alkenyl, Hydroxy oder Nitro,
in Methylenchlorid, um eine Verbindung der Formel (IV) zu bilden;
(b) Acylierung der resultierenden Verbindung der Formel (IV) 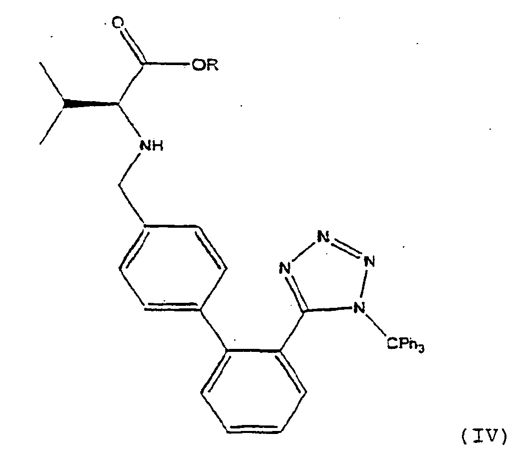 worin R die obige Bedeutung hat, oder eines Salzes davon mit einer Verbindung der Formel (V) 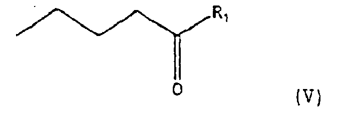 worin R₁ eine Aktivierungsgruppe ist;
(c) Entfernung der Schutzgruppe(n) in der resultierenden Verbindung der Formel (VI) 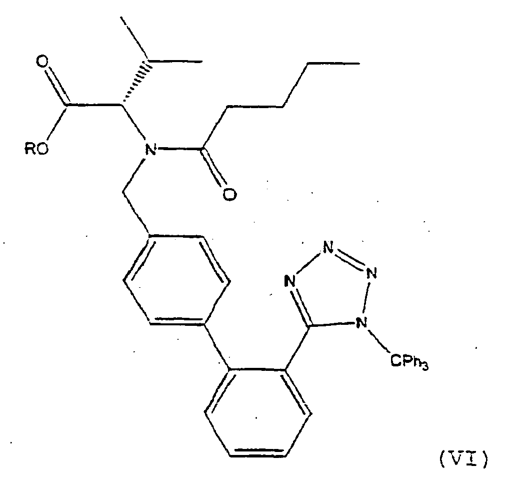 oder einem Salz davon, worin R die obige Bedeutung hat; und
(d) Isolierung der Verbindung der Formel (I), d.h. Valsartan, oder eines pharmazeutisch akzeptablen Salzes davon, und
(e) wenn gewünscht, Formulierung von Valsartan oder seines pharmazeutisch akzeptablen Salzes in eine pharmazeutische Formulierung mittels herkömmlicher Verfahren.

2. Verfahren gemäß Anspruch 1, worin R Methyl ist und die Aktivierungsgruppe R₁ Chlor ist.

3. Verfahren gemäß einem der vorhergehenden Ansprüche, worin die Verbindung (IV) in Schritt (a) als Hydrochlorid isoliert wird.

4. Verfahren gemäß einem der vorhergehenden Ansprüche, worin Schritt (a) in Gegenwart eines Überschusses der Verbindung (III) oder eines Salzes davon durchgeführt wird und nicht verbrauchte Verbindung (III) regeneriert wird.

5. Verfahren gemäß einem der vorhergehenden Ansprüche, worin Schritt (b) in einem Lösungsmittel durchgeführt wird, ausgewählt aus THF, t-Butylmethylether, Methylenchlorid und Isopropylacetat.

6. Verfahren gemäß Anspruch 5, worin das Lösungsmittel THF ist.

7. Verfahren gemäß einem der vorhergehenden Ansprüche, worin Schritt (b) in Gegenwart einer basischen Substanz, ausgewählt aus Pyridin, Dimethylaminopyridin (DMAP) und Triethylamin (TEA), durchgeführt wird.

8. Verfahren gemäß einem der vorhergehenden Ansprüche, worin die Reaktionszeit von Schritt (b) 2 Stunden oder weniger beträgt und die Reaktion anschließend mit Methanol oder Ethanol gequencht wird.

9. Verfahren gemäß einem der vorhergehenden Ansprüche, worin die Entfernung der CPh₃-Gruppe in Schritt (c) in einem Alkohol durch die Zugabe einer Mineralbase bei einer Temperatur von Raumtemperatur bis zur Rückflusstemperatur der Reaktionslösung bewirkt wird.

10. Verfahren gemäß Anspruch 1, worin
- R Methyl oder Benzyl ist,
- die Aktivierungsgruppe Chlor ist
- das Lösungsmittel in Schritt (b) THF ist,
- Schritt (b) in Gegenwart einer basischen Substanz, ausgewählt aus Pyridin, Dimethylaminopyridin (DMAP) und Triethylamin (TEA) durchgeführt wird,
- in Schritt (c) die Entfernung der CPh₃-Gruppe in Schritt (c) in einem Alkohol durch die Zugabe einer Mineralbase bei einer Temperatur von Raumtemperatur bis Rückflusstemperatur der Reaktionslösung bewirkt wird.

11. Verfahren gemäß einem der vorhergehenden Ansprüche, worin im Schritt (d) Valsartan oder sein pharmazeutisch akzeptables Salz in einer Form, ausgewählt aus (i) einer amorphen Form, (ii) einer kristallinen Form oder (iii) einer kristallinen Mischform, isoliert wird.

12. Verfahren gemäß Anspruch 11, **dadurch gekennzeichnet, dass** Valsartan oder ein pharmazeutisch akzeptables Salz davon mittels Evaporation des organischen Lösungsmittels aus einer Valsarten-haltigen Lösung in seiner amorphen Form isoliert wird.

13. Verfahren gemäß Anspruch 11, **dadurch gekennzeichnet, dass** Valsarten oder ein pharmazeutisch akzeptables Salz davon mittels Sprühtrocknung der Valsartan-haltigen Lösung aus einer Valsartan-haltigen Lösung in seiner amorphen Form isoliert wird.

14. Verfahren gemäß Anspruch 12 oder Anspruch 13, worin im Anschluss an die Evaporation oder den Sprühtrocknungsschritt Valsartan oder das pharmazeutische akzeptable Salz davon erneut geschmolzen und abgekühlt wird, wahlweise gefolgt vom Vermahlen der abgekühlten Substanz, um die amorphe Form von Valsartan zu erhalten.

15. Verfahren gemäß Anspruch 11, umfassend den Schritt der Isolierung von Valsartan oder seines pharmazeutisch akzeptablen Salzes in einer amorphen Form, einer kristallinen Form oder einer kristallinen Mischform aus einem Lösungsmittel, ausgewählt aus Methylacetat, Ethylacetat, Ethylformiat, Propylacetat, Isopropylacetat, Propylformiat, Isopropylformiat, Isobutylacetat, Methylenchlorid, Chloroform oder Mischungen davon, wahlweise in Mischung mit einem apolaren Lösungsmittel, ausgewählt aus Hexan, Heptan, Cyclohexan, Toluol, Anisol oder Petrolether.

16. Verfahren gemäß Anspruch 12 oder 15, das nach dem Isolierungsschritt/den Isolierungsschritten ferner die Schritte
(a) Trocknen von Valsartan oder seines pharmazeutisch akzeptablen Salzes bei Raumtemperatur,
(b) während oder nach Schritt (a) Vermahlen oder Granulieren der Substanz bis zu einer durchschnittlichen Partikelgröße von 100 µm oder weniger,
(c) Trocknen der Substanz bei erhöhter Temperatur unter verringertem Druck bis zu einem Lösungsmittelgehalt von 5.000 ppm oder weniger,
umfasst.

17. Verfahren gemäß Anspruch 16, worin das Lösungsmittel Ethylacetat ist.

18. Verfahren gemäß Anspruch 16 oder 17, worin mindestens eine der folgenden Bedingungen zutrifft:
- der Lösungsmittelgehalt nach Schritt (a) beträgt zwischen 5 und 20 Gew.%,
- die Partikelgrößenverteilung am Ende von Schritt (b) ist so eng, dass die Zahl der Partikel mit einem Durchmesser von mehr als 150 µm minimal ist,
- die Trocknungstemperatur in Schritt (c) beträgt zwischen 40 und 60°C,
- die Trocknungszeit in Schritt (c) beträgt 36 Stunden oder weniger, vorzugsweise 24 Stunden oder weniger,
- der Druck in Schritt (c) beträgt 150 hPa oder weniger, am stärksten bevorzugt zwischen 40 und 100 hPa,
- der Lösungsmittelgehalt am Ende von Schritt (c) beträgt weniger als 5.000 ppm.

19. Verbindung der Formel (IV) oder ein Salz davon, worin R Wasserstoff ist

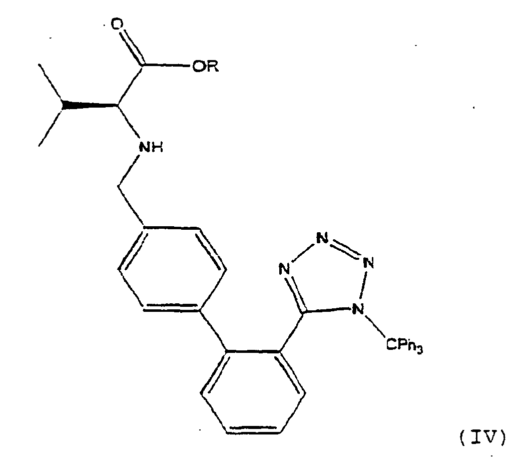

20. Fest Verbindung, die das Hydrochlorid der Verbindung der Formel (IV) darstellt, worin R Methyl ist

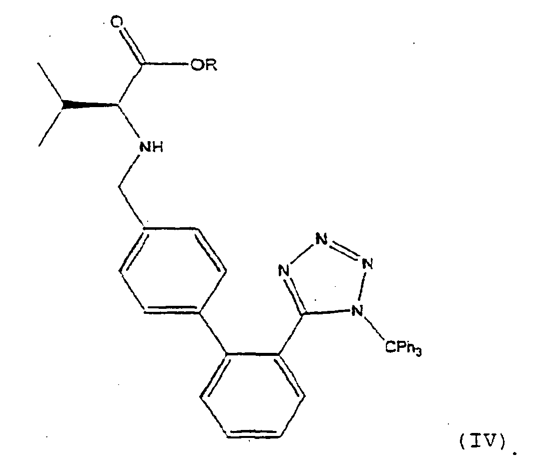

21. Verwendung einer Verbindung gemäß einem der Ansprüche 19 oder 20 für die Synthese von Valsartan oder eines pharmazeutisch akzeptablen Salzes davon.

## Revendications

1. Procédé de préparation de valsartan ayant la formule (I) suivante ou d'un sel pharmaceutiquement acceptable de celui-ci, ou d'une préparation pharmaceutique contenant l'une ou l'autre entité 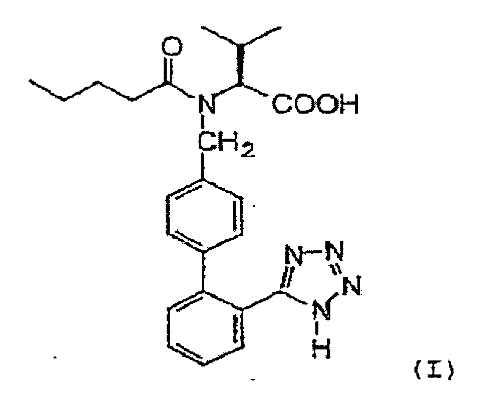 lequel procédé comprend les étapes suivantes :
(a) la réaction d'un composé de formule (II) 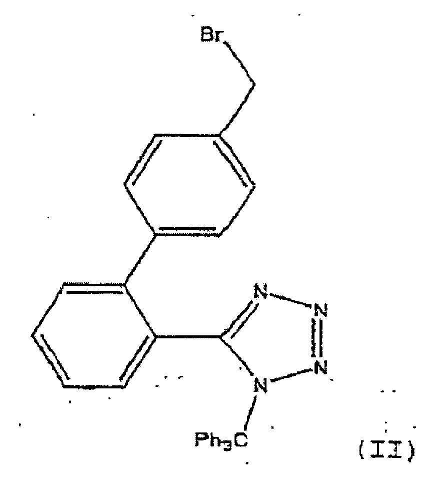 ou d'un sel de celui-ci, avec un composé de formule (III) 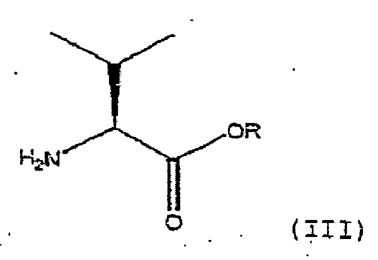 ou un sel de celui-ci, dans lequel R est choisi parmi le groupe consistant en l'hydrogène, un alkyle en C₁-C₅, un alcényle en C₂-C₅ et un benzyle qui peut être facultativement substitué avec un à trois substituants choisis parmi des atomes d'halogène, un alkyle en C₁-C₅, un alcényle en C₂-C₅, un hydroxy, ou un nitro pour former un composé de formule (IV)
dans du chlorure de méthylène ;
(b) l'acylation du composé résultant de formule (IV) 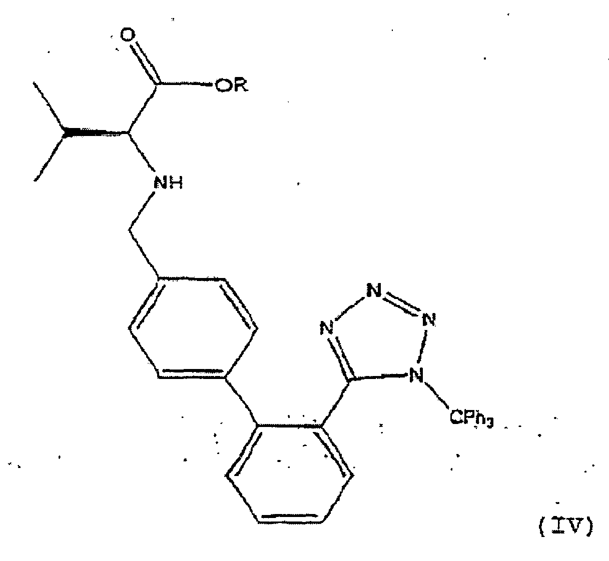 dans lequel R a le sens ci-dessus, ou d'un sel de
celui-ci avec un composé de formule (V) 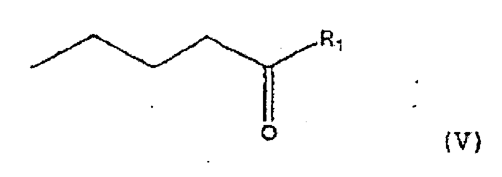 dans lequel R₁ est un groupe d'activation ;
(c) l'élimination du (des) groupe(s) de protection dans le composé résultant de formule (VI) 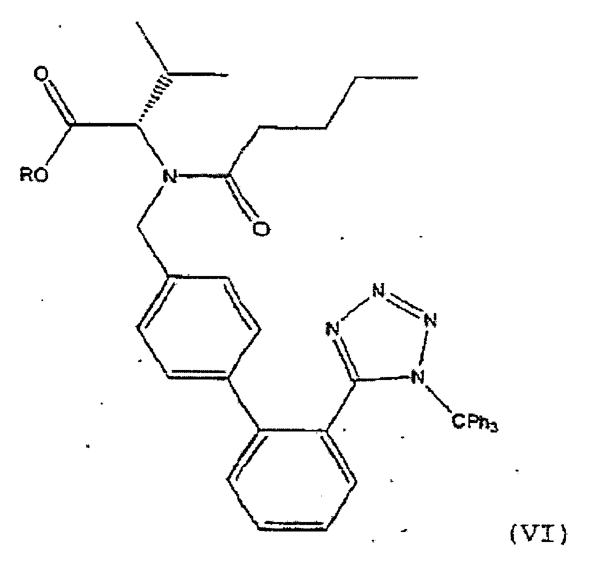 ou un sel de celui-ci, dans lequel R a le sens ci-dessus ; et
(d) l'isolation du composé de formule (I), à savoir le valsartan, ou d'un sel pharmaceutiquement acceptable de celui-ci, et
(e) si tel est désiré, la formulation du valsartan ou de son sel pharmaceutiquement acceptable dans une formulation pharmaceutique par des moyens classiques.

2. Procédé selon la revendication 1 dans lequel R est un méthyle et le groupe d'activation R₁ est le chlore.

3. Procédé selon l'une quelconque des revendications précédentes dans lequel le composé (IV) est isolé à l'étape (a) comme un chlorhydrate.

4. Procédé selon l'une quelconque des revendications précédentes dans lequel l'étape (a) est mise en oeuvre en présence d'un excès de composé (III) ou d'un sel de celui-ci, et le composé (III) non utilisé est régénéré.

5. Procédé selon l'une quelconque des revendications précédentes dans lequel l'étape (b) est mise en oeuvre dans un solvant choisi parmi le THF, l'éther t-butylméthylique, le chlorure de méthylène et l'acétate d'isopropyle.

6. Procédé selon la revendication 5, dans lequel le solvant est le THF.

7. Procédé selon l'une quelconque des revendications précédentes dans lequel l'étape (b) est mise en oeuvre en présence d'une substance basique choisie parmi la pyridine, la diméthylaminopyridine (DMAP) et la triéthylamine (TEA).

8. Procédé selon l'une quelconque des revendications précédentes dans lequel la durée de réaction de l'étape (b) est 2 heures ou moins et la réaction est ensuite interrompue avec du méthanol ou de l'éthanol.

9. Procédé selon l'une quelconque des revendications précédentes dans lequel l'élimination du groupe CPh₃ à l'étape (c) est effectuée dans un alcool avec l'ajout d'une base minérale à une température allant de la température ambiante à la température de reflux de la solution réactionnelle.

10. Procédé selon la revendication 1 dans lequel
- R est un méthyle ou un benzyle,
- le groupe d'activation est le chlore,
- le solvant à l'étape (b) est le THF,
- l'étape (b) est mise en oeuvre en présence d'une substance basique choisie parmi la pyridine, la diméthylaminopyridine (DMAP) et la triéthylamine (TEA),
- à l'étape (c), l'élimination du groupe CPh₃ est effectuée dans un alcool avec l'ajout d'une base minérale à une température allant de la température ambiante à la température de reflux de la solution réactionnelle.

11. Procédé selon l'une quelconque des revendications précédentes dans lequel à l'étape (d), le valsartan ou son sel pharmaceutiquement acceptable est isolé sous une forme choisie parmi (i) une forme amorphe, (ii) une forme cristalline ou (iii) une forme cristalline mixte.

12. Procédé selon la revendication 11 **caractérisé en ce que** le valsartan ou un sel pharmaceutiquement acceptable de celui-ci est isolé à partir d'une solution contenant du valsartan sous sa forme amorphe par évaporation du solvant organique.

13. Procédé selon la revendication 11 **caractérisé en ce que** le valsartan ou un sel pharmaceutiquement acceptable de celui-ci est isolé à partir d'une solution contenant du valsartan sous sa forme amorphe par séchage par pulvérisation de la solution contenant du valsartan.

14. Procédé selon la revendication 12 ou la revendication 13 dans lequel, suite à l'étape d'évaporation ou de séchage par pulvérisation, le valsartan ou le sel pharmaceutiquement acceptable de celui-ci est fondu et refroidi à nouveau, facultativement suivi d'un broyage de la substance refroidie pour donner la forme amorphe du valsartan.

15. Procédé selon la revendication 11 comprenant l'étape d'isolation du valsartan ou de son sel pharmaceutiquement acceptable sous une forme amorphe, cristalline ou cristalline mixte d'un solvant choisi parmi l'acétate de méthyle, l'acétate d'éthyle, le formate d'éthyle, l'acétate de propyle, l'acétate d'isopropyle, le formate de propyle, le formate d'isopropyle, l'acétate d'isobutyle, le chlorure de méthylène, le chloroforme ou des mélanges de ceux-ci, facultativement en mélange avec un solvant apolaire choisi parmi l'hexane, l'heptane, le cyclohexane, le toluène, l'anisole ou l'éther de pétrole.

16. Procédé selon la revendication 12 ou 15 qui, après l'étape (les étapes) d'isolation, comprend en outre les étapes de
(a) séchage du valsartan ou de son sel pharmaceutiquement acceptable à la température ambiante,
(b) pendant ou après l'étape (a), broyage ou granulation de la substance à une taille moyenne de particules de 100 µm ou moins,
(c) séchage de la substance à une température élevée sous pression réduite jusqu'à une teneur en solvant de 5 000 ppm ou moins.

17. Procédé selon la revendication 16 dans lequel le solvant est l'acétate d'éthyle.

18. Procédé selon la revendication 16 ou 17 dans lequel au moins l'une des conditions suivantes s'applique :
- la teneur en solvant après l'étape (a) est entre 5 et 20% en poids,
- la distribution de tailles de particules à la fin de l'étape (b) est si étroite que le nombre de particules ayant un diamètre de plus de 150 µm est minimal,
- la température de séchage à l'étape (c) est entre 40 et 60°C,
- la durée de séchage à l'étape (c) est 36 h ou moins, préférablement 24 h ou moins,
- la pression à l'étape (c) est 150 hPa ou moins, le plus préférablement entre 40 et 100 hPa,
- la teneur en solvant à la fin de l'étape (c) est inférieure à 5 000 ppm.

19. Composé de formule (IV) ou sel de celui-ci, dans lequel R est un hydrogène

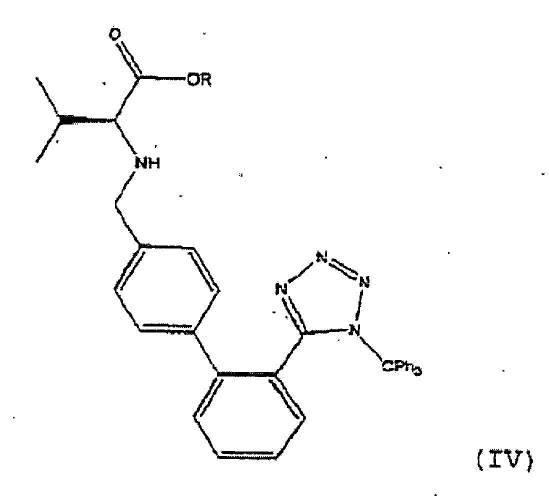

20. Composé solide représentant le chlorhydrate du composé de formule (IV), dans lequel R est un méthyle

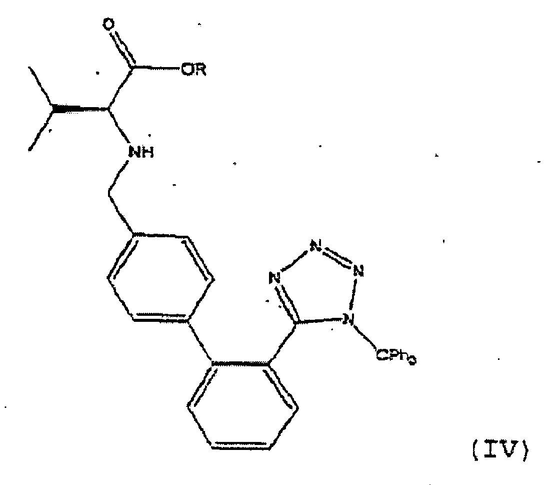

21. Utilisation d'un composé selon l'une quelconque des revendications 19 ou 20 pour la synthèse de valsartan ou d'un sel pharmaceutiquement acceptable de celui-ci.
